# EUROPEAN PATENT SPECIFICATION

(11) **EP 3 417 846 B1**
(45) Date of publication and mention of the grant of the patent: **14.10.2026**
(21) Application number: 17425064.7
(22) Date of filing: 19.06.2017
(51) Int. Cl.: A23L 33/105, A23L 33/12, A61K 9/14

(54) **FOOD AND/OR NUTRACEUTICAL COMPOSITION**
NAHRUNGSMITTEL UND/ODER NUTRAZEUTISCHE ZUSAMMENSETZUNG
ALIMENT ET/OU COMPOSITION NUTRACEUTIQUE

(43) Date of publication of application: 26.12.2018
(73) Proprietor: GIFAR SRL, 42014 Castellarano (RE) (IT)
(72) Inventor: GARITO, Antonio, I-42014 Castellarano (RE) (IT); LEO, Eliana Grazia, I-41124 Modena (IT)
(74) Representative: Ing. C. Corradini & C. S.R.L.

(56) References cited:
- EP-A1- 2 921 167
- EP-A1- 2 985 037
- EP-A1- 3 130 336
- EP-B1- 2 475 352
- WO-A1-2016/146453
- WO-A1-2016/183134
- WO-A1-2017/025588
- WO-A2-00/16756
- JP-A- 2015 155 412
- US-A1- 2015 025 104

## Description

### TECHNICAL FIELD

The present invention relates to a food and/or nutraceutical composition, which comprises an endocannabinoid, preferably but not limited to, Palmitoylethanolamide (PEA).

### PRIOR ART

The use of endocannabinoids for making food and/or nutraceutical and/or pharmacological preparations which mainly exploit the anti-inflammatory properties of such endocannabinoids, in particular of PEA, is known.

Patent EP2475352 shows a known example of a pharmaceutical composition containing PEA in ultramicronized form. WO2016183134A1, WO2016146453A1, JP2015155412A, EP2921167A1, EP2985037A1, EP3130336A1, WO0016756A2, US2015025104A1 and WO2017025588A1 disclose other known compositions containing PEA. Again, WO2017025588A1 discloses a composition containing PEA according the preamble of claim used for ophthalmological treatments.

A need felt in the field, therefore, is to increase and enhance the effects of endocannabinoids, or enhance and increase the anti-inflammatory effects thereof as well as assist that enhanced effect with further beneficial effects, in the manufacture of an edible preparation containing a food and/or nutraceutical composition that is particularly effective, usable in a simple manner and easy assimilated by the body.

One aim of the present invention is to meet such needs of the prior art.

These aims are achieved by the features of the invention described in each independent claim. The dependent claims outline preferred and/or particularly advantageous aspects of the invention.

### DESCRIPTION OF THE INVENTION

The present invention provides a food and/or nutraceutical composition in the form of a micro-particle lipo-complex having a particle size comprised between 20 nm and 80 µm. The composition comprises an endocannabinoid, an active ingredient, and an excipient, wherein the excipient is an emulsifier. The micro-particle lipo-complex comprises the endocannabinoid and the active ingredient associated together in a lipid complex stabilized by the emulsifying excipient. The active ingredient is selected from a group consisting of at least one cannabinoid, including cannabidiol (CBD), cannabigerol (CBG), cannabitriol (CBT), cannabigerolic acid (CBGA), cannabivarin (CBV), cannabinol (CBN), cannabichromene (CBC), cannabidivarin (CBDV), cannabidiolic acid (CBDA) and tetrahydrocannabinol (THC), or a mixture thereof.

Thanks to the combination of the active ingredient with an endocannabinoid, it has been studied that the physiological anti-inflammatory effect of the endocannabinoid is enhanced and it is possible to combine it with new effects of the active ingredient also enhanced by the synergistic effect with the endocannabinoid.

Preferably, the endocannabinoid may be selected from the group consisting of: N-Palmitoylethanolamide (N-palmitoylethanolamine or PEA), 2-arachidonoyl-glycerol (2-AG), Arachidonoylethanolamide (N-arachidonoyl-ethanolamine) (ANANDAMIDE), 2-arachidonyl glyceryl ether (NOLADIN ETHER), N-arachidonyl-dopamine (NADA), O-arachidonoyl-ethanolamine (VIRODHAMINE), Oleamide, N-stearoyl-1-oleoyl-2-palmitoyl-3-phosphatidylethanolamine, N-arachidonoyl-phosphatidylethanolamine (NArPE), N-oleoylethano-lamine(Oleoylethanolamide) (OEA), 2-linoleoylglycerol, 2-oleoylglycerol, N-Acyltaurine (N-Acetyltaurine), N-acylglicine (N-Acetylglicine), N-Acyldopamine (N-Acetyldopamine), and N-stearylethanolamine (SEA) or a mixture thereof. Advantageously, the endocannabinoid is N-Palmitoylethanolamide (PEA). Advantageously, the excipient is selected from the group consisting of a lipid, in particular lecithin, cholesterol, phosphatidylcholine, phosphatidylethanolamine and phosphatidylserine.

The excipient may also be selected from the group consisting of a viscosizing substance such as: natural gums, natural saponins free from aglycone or conjugated with the aglycone, and natural gelatines, in particular arabic gum, Greek hay (50% dry extract in saponins) and gelatin.

The above composition is in the form of a micro-particle lipo-complex (where the root "lipo" means any combination of so-called lipophilic substances), i.e. it has a stable structure of a lipo-complex, in which the endocannabinoid and the active ingredient are associated together, also thanks to the stabilizing action of a suitable emulsifying excipient. The endocannabinoid, insoluble in water and non-dispersible, in the presence of the emulsifying excipient and an active ingredient (following a suitable method described hereinafter), surprisingly exponentially increases its dispersibility and thus its potential bioavailability.

The endocannibinoid, preferably, may be present in a percentage by weight with respect to the weight of the composition of between 15% and 90%, preferably between 36% and 52%.

The active ingredient may be present in a percentage by weight with respect to the weight of the composition of between 0.1% and 80%, preferably between 10% and 40%, even more preferably between 15% and 26%.

A preferred composition may consist of lipo-complexes consisting of PEA, Cannabidiol and Lecithin.

A further example is defined by a composition which may consist of lipo-complexes consisting of PEA, Cholesterol and Cannabidiol.

The composition is in the form of nano-particles or micro-particles having particle size comprised between 20 nm and 80 µm.

Below there are listed the main additional physiological and synergistic effects with respect to the anti-inflammatory effect of the endocannabinoid alone of the composition among an endocannabinoid, such as PEA, and some of the most significant active ingredients among those listed above.

Composition containing PEA and a cannabinoid (CBD), in which the physiological effect brought by the cannabinoid is pain relieving/analgesic. Composition, not according to the present invention, containing PEA and Boswellic acid, in which the physiological effect brought by Boswellic acid is analgesic/pain relieving.

Composition, not according to the present invention, containing PEA and Curcumin, in which the physiological effect brought by Curcumin is antioxidant.

### BEST MODE OF CARRYING OUT THE INVENTION

The composition of the invention is in the form of a micro-particle lipo-complex of nano-particles or micro-particles having particle size comprised between 20 nm and 80 µm, the composition comprising: an endocannabinoid, an active ingredient and an excipient, wherein the excipient is an emulsifier, wherein the micro-particle lipo-complex comprises the endocannabinoid and the active ingredient associated together in a lipid complex stabilized by the emulsifying excipient, and wherein the active ingredient is selected in - a group consisting of at least one cannabinoid, including cannabidiol (CBD), cannabigerol (CBG), cannabitriol (CBT), cannabigerolic acid (CBGA), cannabivarin (CBV), cannabinol (CBN), cannabichromene (CBC), cannabidivarin (CBDV), cannabidiolic acid (CBDA) and tetrahydrocannabinol (THC) or a mixture thereof.

In the light of the above, the method for making a composition as described above in the form of a lipo-complex is as follows.

The method provides the steps of:
- preparing an excipient wherein the excipient is an emulsifier, an endocannabinoid and an active ingredient selected in the group consisting of at least one cannabinoid, including cannabidiol (CBD), cannabigerol (CBG), cannabitriol (CBT), cannabigerolic acid (CBGA), cannabivarin (CBV), cannabinol (CBN), cannabichromene (CBC), cannabidivarin (CBDV), cannabidiolic acid (CBDA) and tetrahydrocannabinol (THC) or a mixture thereof;
- dissolving and mixing the same in a solvent;
- removing the solvent, thereby forming a lipid precipitate a micro-particle lipo-complex, wherein the lipid precipitate is dry and in powder form having particle size comprised between 20 nm and 80 µm.

A first method used, called solvent injection method, allows forming micro-precipitates.

**In** this case, a dosed amount of endocannabinoid, active ingredient and excipient is dissolved in the solvent (organic, for example consisting of alcohols, acetone, acetonitrile or any solvent that can be mixed with water). This solution is injected through a syringe/injector in a container containing an aqueous phase (water) and an optional further excipient (such as for example a viscosizing excipient, like Greek hay and/or gelatin or the like) and after (24 hours) evaporation of the solvent, a suspension of micro-particle lipo-complexes is obtained which are then recovered, for example by filtration or lyophilization.

A second method used, called emulsion method with solvent evaporation, allows obtaining particles of the desired size.

In this case, an emulsion (O/W) is formed between the solvent (organic, for example consisting of chloroform, dichloromethane or other organic solvent immiscible with water possibly mixed with alcohol), in which a dosed amount of a mixture of endocannabinoid, active ingredient and excipient is dissolved, and an external aqueous phase (in which the aqueous phase comprises water and a possible further excipient, such as a viscosizing excipient, such as Greek hay and/or gelatin or the like). After the evaporation of the solvent, a suspension of micro-particle lipo-complexes is obtained that are then recovered for example by filtration or lyophilization.

In general, the solvent is an organic lipophilic solvent, for example a halogenated hydrocarbon, such as dichloromethane or chloroform or ethanol, or similar solvents or mixtures thereof.

**Lipo-complex of Pea with Boswellic acids** (Examples not according to the invention)

### Example 1 (obtained by the emulsion/solvent evaporation method)

To a solvent consisting of an equimolar mixture of ethanol and chloroform is added and dissolved a (dry) mixture consisting of PEA, Boswellia (Serrata) extract, comprising at least 50% Boswellic acids and (Soy) Lecithin comprising at least 45% Phosphatidylcholine.

This mixture has the following formulation:
PEA 36%;
Boswellia Extract 18%;
Soy lecithin 46%;
wherein all the percentages are by weight with respect to the weight of the added mixture.

The solvent is in the suitable volume (minimum) to dissolve the entire volume of dry mixture (up to the removal of any lumps into the same).

The dissolution of the mixture in the solvent is promoted by stirring, such as that obtained by means of a stirrer (Vortex, for example).

Subsequently, the composition dissolved in the solvent was placed in an aqueous phase, for example previously formed.

The aqueous phase comprises water and a further excipient, such as for example Greek hay (for example, in a percentage by weight substantially equal to 0.5% with respect to the total (dry) composition).

The amount of water is the minimum amount necessary to dissolve the complex.

An emulsion is then formed using a homogenizer/disperser like Ultraturrax, at a minimum speed of 24,000 rpm for a total of at least 3 minutes.

The emulsion is immediately left in evaporation through a blade stirrer at an exemplary speed of 1,700 rpm and for a duration of minimum 3 hours.

The obtained suspension is allowed to freeze to be then freeze-dried in a crystallizer for a period of 24h (thus removing the solvent) to obtain a particle precipitate.

With the removal of water and solvent, solid particles are obtained, i.e. the micro-particle lipo-complexes that have average sizes of around 20 ± 5 micrometers (µm).

### Example 2 (obtained by the solvent injection method)

To a solvent consisting of ethanol is added and dissolved a (dry) mixture consisting of PEA, Boswellia (Serrata) extract, comprising at least 50% Boswellic acids and Cholesterol.

This mixture has the following formulation:
PEA 51%;
Boswellia Extract 26%;
Cholesterol 23%,
wherein all the percentages are by weight with respect to the weight of the added mixture.

The solvent is in the suitable volume (minimum) to dissolve the entire volume of dry mixture (up to the removal of any lumps into the same).

The dissolution of the mixture in the solvent is promoted by stirring, such as that obtained by means of a stirrer (Vortex, for example).

Subsequently, the composition dissolved in the solvent was placed in an aqueous phase (for example previously formed), for example by injection with an injector (syringe).

The aqueous phase comprises water and a further excipient, such as for example gelatin (for example, in a percentage by weight substantially equal to 2% with respect to the total (dry) composition).

The amount of water is the minimum amount necessary to dissolve the complex.

Subsequently, the solvent (and water) were removed, for example by means of an evaporator to obtain a particle precipitate.

With the evaporation of water and solvent, solid particles are obtained, i.e. the micro-particle lipo-complexes that have average sizes of around 44 ± 5 micrometers (µm).

### Lipo-complex of Pea with Cannabidiol (CBD)

### Example 1 (obtained by the emulsion/solvent evaporation method)

To a solvent consisting of an equimolar mixture of ethanol and chloroform is added and dissolved a (dry) mixture consisting of PEA, Cannabis extract, comprising at least 50% Cannabidiol and (Soy) Lecithin comprising at least 45% Phosphatidylcholine.

This mixture has the following formulation:
PEA 36%;
Cannabis extract 18%;
Soy Lecithin 46%;
wherein all the percentages are by weight with respect to the weight of the added mixture.

The solvent is in the suitable volume (minimum) to dissolve the entire volume of dry mixture (up to the removal of any lumps into the same).

The dissolution of the mixture in the solvent is promoted by stirring, such as that obtained by means of a stirrer (Vortex, for example).

Subsequently, the composition dissolved in the solvent was placed in an aqueous phase, for example previously formed.

The aqueous phase comprises water and a further excipient, such as for example Greek hay (for example, in a percentage by weight substantially equal to 0.5% with respect to the total (dry) composition).

The amount of water is the minimum amount necessary to dissolve the complex.

An emulsion is then formed using a homogenizer/disperser like Ultraturrax, at a minimum speed of 24,000 rpm for a total of at least 3 minutes.

The emulsion is immediately left in evaporation through a blade stirrer at an exemplary speed of 1,700 rpm and for a duration of minimum 3 hours.

The obtained suspension is allowed to freeze to be then freeze-dried in a crystallizer for a period of 24h (thus removing the solvent) to obtain a particle precipitate.

With the removal of water and solvent, solid particles are obtained, i.e. the micro-particle lipo-complexes that have average sizes of around 70 ± 5 micrometers (µm).

If in addition to the Greek hay also (2%) gelatin is included, particles of 45 ± 5 micrometers (µm) are obtained.

### Example 2 (obtained by the solvent injection method)

To a solvent consisting of ethanol is added and dissolved a (dry) mixture consisting of PEA, Cannabis extract, comprising at least 50% Cannabidiol and Cholesterol.

This mixture has the following formulation:
PEA 51%;
Cannabis extract 26%;
Cholesterol 23%,
wherein all the percentages are by weight with respect to the weight of the added mixture.

The solvent is in the suitable volume (minimum) to dissolve the entire volume of dry mixture (up to the removal of any lumps into the same).

The dissolution of the mixture in the solvent is promoted by stirring, such as that obtained by means of a stirrer (Vortex, for example).

Subsequently, the composition dissolved in the solvent was placed in an aqueous phase, for example previously formed, for example by injection with an injector (syringe).

The aqueous phase comprises water and a further excipient, such as for example gelatin (for example, in a percentage by weight substantially equal to 2% with respect to the total (dry) composition).

The amount of water is the minimum amount necessary to dissolve the complex.

Subsequently, the solvent (and water) were removed, for example by means of an evaporator to obtain a particle precipitate.

With the evaporation of water and solvent, solid particles are obtained, i.e. the micro-particle lipo-complexes that have average sizes of around 45 ± 5 micrometers (µm).

**Lipo-complex of Pea** (Examples not according to the invention)

### Example 1 (obtained by the emulsion/solvent evaporation method)

To a solvent consisting of an equimolar mixture of ethanol and chloroform is added and dissolved a (dry) mixture consisting of PEA and (Soy) Lecithin comprising at least 45% Phosphatidylcholine.

This mixture has the following formulation:
PEA 43.75%;
Soy Lecithin 56.25%;
wherein all the percentages are by weight with respect to the weight of the added mixture.

The solvent is in the suitable volume (minimum) to dissolve the entire volume of dry mixture (up to the removal of any lumps into the same).

The dissolution of the mixture in the solvent is promoted by stirring, such as that obtained by means of a stirrer (Vortex, for example).

Subsequently, the composition dissolved in the solvent was placed in an aqueous phase, for example previously formed.

The aqueous phase comprises water and a further excipient, such as for example Greek hay (for example, in a percentage by weight substantially equal to 0.5% with respect to the total (dry) composition).

The amount of water is the minimum amount necessary to dissolve the complex.

An emulsion is then formed using a homogenizer/disperser like Ultraturrax, at a minimum speed of 24,000 rpm for a total of at least 3 minutes.

The emulsion is immediately left in evaporation through a blade stirrer at an exemplary speed of 1,700 rpm and for a duration of minimum 3 hours.

The obtained suspension is allowed to freeze to be then freeze-dried in a crystallizer for a period of 24h (thus removing the solvent) to obtain a particle precipitate.

With the removal of water and solvent, solid particles are obtained, i.e. the micro-particle lipo-complexes that have average sizes of around 20 ± 5 micrometers (µm).

If in addition to the Greek hay also (2%) gelatin is included, particles of 25 ± 5 micrometers (µm) are obtained.

### Example 2 (obtained by the solvent injection method)

To a solvent consisting of ethanol is added and dissolved a (dry) mixture consisting of PEA and Cholesterol.

This mixture has the following formulation:
PEA 70%;
Cholesterol 30%,
wherein all the percentages are by weight with respect to the weight of the added mixture.

The solvent is in the suitable volume (minimum) to dissolve the entire volume of dry mixture (up to the removal of any lumps into the same).

The dissolution of the mixture in the solvent is promoted by stirring, such as that obtained by means of a stirrer (Vortex, for example).

Subsequently, the composition dissolved in the solvent was placed in an aqueous phase, for example previously formed, for example by injection with an injector (syringe).

The aqueous phase comprises water and a further excipient, such as for example gelatin (for example, in a percentage by weight substantially equal to 2% with respect to the total (dry) composition).

The amount of water is the minimum amount necessary to dissolve the complex.

Subsequently, the solvent (and water) are removed, for example by means of an evaporator to obtain a particle precipitate.

With the evaporation of water and solvent, solid particles are obtained, i.e. the micro-particle lipo-complexes that have average sizes of around 23 ± 5 micrometers (µm).

## Claims

1. A food and/or nutraceutical composition in the form of a micro-particle lipo-complex of nano-particles or micro-particles having particle size comprised between 20 nm and 80 µm, the composition comprising: an endocannabinoid, an active ingredient and an excipient, wherein the excipient is an emulsifier, wherein the micro-particle lipo-complex comprises the endocannabinoid and the active ingredient associated together in a lipid complex stabilized by the emulsifying excipient, and wherein the active ingredient is selected in
- a group consisting of at least one cannabinoid, including cannabidiol (CBD), cannabigerol (CBG), cannabitriol (CBT), cannabigerolic acid (CBGA), cannabivarin (CBV), cannabinol (CBN), cannabichromene (CBC), cannabidivarin (CBDV), cannabidiolic acid (CBDA) and tetrahydrocannabinol (THC) or a mixture thereof.

2. The composition according to claim 1, wherein the endocannabinoid is selected from the group consisting of: N-Palmitoylethanolamide (N-palmitoylethanolamine or PEA), 2-arachidonoyl-glycerol (2-AG), Arachidonoylethanolamide (N-arachidonoyl-ethanolamine) (ANANDAMIDE), 2-arachidonyl glyceryl ether (NOLADIN ETHER), N-arachidonyl-dopamine (NADA), O-arachidonoyl-ethanolamine (VIRODHAMINE), Oleamide, N-stearoyl-1-oleoyl-2-palmitoyl-3-phosphatidylethanolamine, N-arachidonoyl-phosphatidylethanolamine (NArPE), N-oleoylethanolamine(Oleoylethanolamide) (OEA), 2-linoleoylglycerol, 2-oleoylglycerol, N-Acyltaurine (N-Acetyltaurine), N-acylglicine (N-Acetylglicine), N-Acyldopamine (N-Acetyldopamine), and N-stearylethanolamine (SEA) or a mixture thereof.

3. The composition according to claim 1, wherein the endocannabinoid is N-Palmitoylethanolamide (PEA).

4. The composition according to claim 1, wherein the excipient is selected from the group consisting of a lipid, including lecithin, cholesterol, phosphatidylcholine, phosphatidylethanolamine and phosphatidylserine, or of a viscosizing substance such as natural gums including arabic gum, natural saponins free from aglycone or conjugated with aglycone, including Greek hay, and natural gelatins, including gelatin.

5. The composition according to claim 1, wherein the endocannibinoid is present in a percentage by weight with respect to the weight of the composition of between 15% and 90%, preferably between 36% and 52%.

6. The composition according to claim 1, wherein the active ingredient is present in a percentage by weight with respect to the weight of the composition of between 0.1% and 80%, preferably between 10% and 40%, even more preferably between 15% and 26%.

7. Dietary supplement containing the composition according to any one of the preceding claims in a pre-dosed pharmaceutical form.

8. Process for preparing the composition according to claim 1, comprising the steps of:
- preparing an excipient wherein the excipient is an emulsifier, an endocannabinoid and an active ingredient selected in the group consisting of at least one cannabinoid, including cannabidiol (CBD), cannabigerol (CBG), cannabitriol (CBT), cannabigerolic acid (CBGA), cannabivarin (CBV), cannabinol (CBN), cannabichromene (CBC), cannabidivarin (CBDV), cannabidiolic acid (CBDA) and tetrahydrocannabinol (THC) or a mixture thereof;
- dissolving and mixing the same in a solvent;
- removing the solvent, thereby forming a lipid precipitate in the form of a micro-particle lipo-complex, wherein the lipid precipitate is dry and in powder form having particle size comprised between 20 nm and 80 µm.

## Patentansprüche

1. Lebensmittel- und/oder Nutrazeutika-Zusammensetzung in Form eines Mikropartikel-Lipokomplexes aus Nanopartikeln oder Mikropartikeln, die eine Partikelgröße zwischen 20 nm und 80 µm aufweisen, die Zusammensetzung umfassend: ein Endocannabinoid, einen Wirkstoff und einen Hilfsstoff, wobei der Hilfsstoff ein Emulgator ist, wobei der Mikropartikel-Lipokomplex das Endocannabinoid und den Wirkstoff umfasst, die zusammen in einem Lipidkomplex assoziiert sind, der durch den emulgierenden Hilfsstoff stabilisiert wird, und wobei der Wirkstoff ausgewählt ist aus
- einer Gruppe, bestehend aus mindestens einem Cannabinoid, einschließlich Cannabidiol (CBD), Cannabigerol (CBG), Cannabitriol (CBT), Cannabigerolsäure (CBGA), Cannabivarin (CBV), Cannabinol (CBN), Cannabichromen (CBC), Cannabidivarin (CBDV), Cannabidiolsäure (CBDA) und Tetrahydrocannabinol (THC) oder einem Gemisch davon.

2. Zusammensetzung nach Anspruch 1, wobei das Endocannabinoid ausgewählt ist aus der Gruppe, bestehend aus: N-Palmitoylethanolamid (N-Palmitoylethanolamin oder PEA), 2-Arachidonoylglycerin (2-AG), Arachidonoylethanolamid (N-Arachidonoylethanolamin) (ANANDAMID), 2-Arachidonoylglycerylether (NOLADIN-ETHER), N-Arachidonoyl-Dopamin (NADA), O-Arachidonoylethanolamin (VIRODHAMIN), Oleamid, N-Stearoyl-1-oleoyl-2-palmitoyl-3-phosphatidylethanolamin, N-Arachidonoyl-phosphatidylethanolamin (NArPE), N-Oleoylethanolamin (Oleoylethanolamid) (OEA), 2-Linoleoylglycerin, 2-Oleoylglycerin, N-Acyltaurin (N-Acetyltaurin), N-Acylglycin (N-Acetylglycin), N-Acyldopamin (N-Acetyldopamin) und N-Stearylethanolamin (SEA) oder einem Gemisch davon.

3. Zusammensetzung nach Anspruch 1, wobei das Endocannabinoid N-Palmitoylethanolamid (PEA) ist.

4. Zusammensetzung nach Anspruch 1, wobei der Hilfsstoff ausgewählt ist aus der Gruppe, bestehend aus einem Lipid, einschließlich Lecithin, Cholesterin, Phosphatidylcholin, Phosphatidylethanolamin und Phosphatidylserin, oder aus einer viskositätserhöhenden Substanz, wie beispielsweise natürlichem Gummi, einschließlich Gummi arabicum, natürlichen Saponinen, die frei von Aglykon sind oder mit Aglykon konjugiert sind, einschließlich Bockshornklee, und natürlichen Gelatinen, einschließlich Gelatine.

5. Zusammensetzung nach Anspruch 1, wobei das Endocannabinoid in einem Massenanteil, bezogen auf das Gewicht der Zusammensetzung, von zwischen 15 % und 90 %, vorzugsweise zwischen 36 % und 52 %, vorhanden ist.

6. Zusammensetzung nach Anspruch 1, wobei der Wirkstoff in einem Massenanteil, bezogen auf das Gewicht der Zusammensetzung, von zwischen 0,1 % und 80 %, vorzugsweise zwischen 10 % und 40 % und noch bevorzugter zwischen 15 % und 26 % vorhanden ist.

7. Nahrungsergänzungsmittel, das die Zusammensetzung nach einem der vorherigen Ansprüche in einer vordosierten Darreichungsform enthält.

8. Verfahren zum Herstellen der Zusammensetzung nach Anspruch 1, umfassend die folgenden Schritte:
- Herstellen eines Hilfsstoffs, wobei der Hilfsstoff ein Emulgator, ein Endocannabinoid und ein Wirkstoff ist, ausgewählt aus der Gruppe bestehend aus mindestens einem Cannabinoid, einschließlich Cannabidiol (CBD), Cannabigerol (CBG), Cannabitriol (CBT), Cannabigerolsäure (CBGA), Cannabivarin (CBV), Cannabinol (CBN), Cannabichromen (CBC), Cannabidivarin (CBDV), Cannabidiolsäure (CBDA) und Tetrahydrocannabinol (THC) oder einem Gemisch davon;
- Auflösen und Mischen desselben in einem Lösungsmittel;
- Entfernen des Lösungsmittels, wodurch ein Lipidpräzipitat in Form eines Mikropartikel-Lipokomplexes gebildet wird, wobei das Lipidpräzipitat trocken und in Pulverform ist, das eine Partikelgröße zwischen 20 nm und 80 µm aufweist.

## Revendications

1. Composition alimentaire et/ou nutraceutique en forme d'un lipo-complexe à microparticules constitué de nanoparticules ou de microparticules dont la taille est comprise entre 20 nm et 80 µm, ladite composition comprenant : un endocannabinoïde, un principe actif et un excipient, dans laquelle l'excipient est un émulsifiant, dans laquelle le lipo-complexe à microparticules comprend l'endocannabinoïde et le principe actif associés l'un à l'autre dans un complexe lipidique stabilisé par l'excipient émulsifiant, et dans laquelle le principe actif est choisi parmi
- un groupe composé d'au moins un cannabinoïde, notamment le cannabidiol (CBD), le cannabigérol (CBG), le cannabitriol (CBT), l'acide cannabigérolique (CBGA), la cannabivarine (CBV), le cannabinol (CBN), le cannabichromène (CBC), la cannabidivarin (CBDV), l'acide cannabidiolique (CBDA) et le tétrahydrocannabinol (THC), ou un mélange de ceux-ci.

2. Composition selon la revendication 1, dans laquelle l'endocannabinoïde est choisi dans le groupe constitué de : N-palmitoyléthanolamide (N-palmitoyléthanolamine ou PEA), 2-arachidonoylglycérol (2-AG), arachidonoyléthanolamide (N-arachidonoyléthanolamine) (ANANDAMIDE), éther glycérylique de 2-arachidonoyle (NOLADIN ETHER), N-arachidonoyl-dopamine (NADA), O-arachidonoyl-éthanolamine (VIRODHAMINE), Oléamide, N-stéaroyl-1-oléoyl-2-palmitoyl-3-phosphatidyléthanolamine, N-arachidonoyl-phosphatidyléthanolamine (NArPE), N-oléoyléthanolamine (oléoyléthanolamide) (OEA), 2-linoléoylglycérol, 2-oléoylglycérol, N-Acyltaurine (N-Acétyltaurine), N-acylglycine (N-Acétylglycine), N-Acyldopamine (N-Acétyldopamine) et N-stéaryléthanolamine (SEA) ou un mélange de ceux-ci.

3. Composition selon la revendication 1, dans laquelle l'endocannabinoïde est N-Palmitoyléthanolamide (PEA).

4. Composition selon la revendication 1, dans laquelle l'excipient est choisi parmi le groupe constitué d'un lipide, notamment la lécithine, le cholestérol, la phosphatidylcholine, la phosphatidyléthanolamine et la phosphatidylsérine, ou d'une substance viscosifiante telle que des gommes naturelles, notamment la gomme arabique, des saponines naturelles exemptes d'aglycone ou conjuguées à de l'aglycone, notamment le foin grec, et des gélatines naturelles, notamment la gélatine.

5. Composition selon la revendication 1, dans laquelle l'endocannabinoïde est présent en un pourcentage en poids, par rapport au poids de la composition, compris entre 15 % et 90 %, de préférence entre 36 % et 52 %.

6. Composition selon la revendication 1, dans laquelle le principe actif est présent en un pourcentage en poids, par rapport au poids de la composition, compris entre 0,1 % et 80 %, de préférence entre 10 % et 40 %, et plus préférablement encore entre 15 % et 26 %.

7. Complément alimentaire contenant la composition selon l'une quelconque des revendications précédentes, sous une forme pharmaceutique prédosée.

8. Procédé de préparation de la composition selon la revendication 1, comprenant les étapes suivantes :
- la préparation d'un excipient, dans lequel l'excipient est un émulsifiant, un endocannabinoïde et un principe actif choisi parmi le groupe comprenant au moins un cannabinoïde, notamment le cannabidiol (CBD), le cannabigérol (CBG), le cannabitriol (CBT), l'acide cannabigérolique (CBGA), la cannabivarine (CBV), le cannabinol (CBN), le cannabichromène (CBC), la cannabidivarin (CBDV), l'acide cannabidiolique (CBDA) et le tétrahydrocannabinol (THC), ou un mélange de ceux-ci ;
- la dissolution et le mélange de ceux-ci dans un solvant ;
- l'élimination du solvant, ce qui entraîne la formation d'un précipité lipidique sous la forme d'un lipo-complexe à microparticules,
dans lequel le précipité lipidique étant sec et se présentant sous forme de poudre dont la taille des particules est comprise entre 20 nm et 80 µm.
